# EUROPEAN PATENT SPECIFICATION

(11) **EP 2 493 377 B1**
(45) Date of publication and mention of the grant of the patent: **25.11.2026**
(21) Application number: 10828911.7
(22) Date of filing: 27.10.2010
(51) Int. Cl.: A61B 5/256, A61B 5/291, A61B 5/00, A61B 5/274, A61B 5/369, A61B 5/271

(54) **HEAD HARNESS & WIRELESS EEG MONITORING SYSTEM**
KOPFGESCHIRR UND DRAHTLOSES EEG-ÜBERWACHUNGSSYSTEM
CASQUE ET SYSTÈME DE SUIVI SANS FIL POUR EEG

(30) Priority: 27.10.2009 US 255343 P
(43) Date of publication of application: 05.09.2012
(73) Proprietor: Neurovigil, Inc., La Jolla, CA 92037 (US)
(72) Inventor: LOW, Philip, La Jolla CA 92037 (US); CHI, Yu, M., La Jolla CA 92092 (US); JOSHI, Siddaharth, La Jolla CA 92122 (US); UEBELHER, Christopher, San Diego CA 92126 (US); DUBOIS, Yvon, A., Escondido CA 92026 (US); LIU, Kevin, Walnut CA 91789 (US)
(74) Representative: Mewburn Ellis LLP
(86) International application number: PCT/US2010/054346
(87) International publication number: WO 2011/056679

(56) References cited:
- EP-A1- 0 541 393
- WO-A2-02/30261
- GB-A- 2 447 354
- KR-A- 20030 002 677
- KR-A- 20070 109 044
- US-A- 3 998 213
- US-A- 4 936 306
- US-A- 5 406 956
- US-A1- 2003 032 893
- US-A1- 2008 082 019
- US-A1- 2009 253 996
- DEGEN T ET AL: "Enhancing Interference Rejection of Preamplified Electrodes by Automated Gain Adaption", IEEE TRANSACTIONS ON BIOMEDICAL ENGINEERING, IEEE SERVICE CENTER, PISCATAWAY, NJ, USA, vol. 51, no. 11, 1 November 2004 (2004-11-01), pages 2031 - 2039, XP011120480, ISSN: 0018-9294, DOI: 10.1109/TBME.2004.834296
- ADHAMI R ET AL: "Stress monitoring using a distributed wireless intelligent sensor system", IEEE ENGINEERING IN MEDICINE AND BIOLOGY MAGAZINE, IEEE SERVICE CENTER, PISACATAWAY, NJ, US, vol. 22, no. 3, 1 May 2003 (2003-05-01), pages 49 - 55, XP011098589, ISSN: 0739-5175

## Description

### FIELD OF THE INVENTION

The present invention relates generally to devices for the acquisition of physiological data. More particularly the present invention is directed to a physiological data acquisition assembly that may be used in combination with a head harness, applied by a user without assistance and that acquires quality physiological data including but not limited to EEG, EKG, EMG, and EOG signals.

### BACKGROUND OF THE INVENTION

An electroencephalogram (EEG) is the graphic record of brain action potentials. The EEG supplies important information about the brain function of a patient. Conventional monitoring and diagnostic equipment is structured in such a way that several electrodes are mounted on the subject, which tap the brain signals and transmit the signals via cables to amplifier units. Normally, separate electrodes are used for each measurement parameter and usually require that numerous electrodes be placed on a patient's head. Proper placement of the electrodes is important and usually follows the International 10/20 System, which is the widely accepted method describing the placement of the electrodes on the patient's head. As a result, EEG data is often gathered in a clinical setting where the electrodes can be properly placed with technical assistance.

Due to the nature of the conventional methods applied in EEG examinations, many cables are suspended on the patient. The cables are troublesome in that they constrict the patient and greatly limit the freedom of movement. In addition, the process of properly placing numerous electrodes is time consuming and often, due to the number of wires and electrodes, will result in ineffective adhesion and loss of contact with the scalp of the patient.

These conventional methods are often a problem in monitoring patients in sleep centers. The complications associated with the cumbersome nature of the conventional detection and recording devices coupled with the subject being in a foreign environment greatly hinders monitoring a patient's sleep stage. The combination of the cables and sleep center environment will have a bearing on the patient's ability to sleep and subsequent results of the exam. If a subject has the ability to be monitored comfortably from home without restricting their freedom of movement, anxiety is reduced therefore producing more accurate results.

There is therefore a need for a new and improved physiological data acquisition system and apparatus that facilitates application of electrodes by the patient without technical assistance and that collects quality physiological data, including but not limited to EEG, EKG, EMG, and EOG signals from a single active electrode.

US5406956A describes headbands for positioning electrodes at preferred locations on a subject's scalp for obtaining electrical responses therefrom.

US2008/082019A1 describes a device comprising a head mounting arrangement for seizure detection.

WO02/30261A2 describes a flexible, conformable, sensor assembly including an electrode array adapted for stable, long-term recording of EEG signals from a pre-term or neonatal infant in intensive care.

### SUMMARY OF THE INVENTION

In a first aspect, there is provided a physiological data acquisition assembly according to claim 1. In a second aspect, there is provided a system for acquiring physiological data, the system comprising the physiological data acquisition assembly according to the first aspect. The system and assembly for acquiring physiological data can be applied by a user without technical assistance and acquire quality physiological data including but not limited to EEG, EKG, EMG, and EOG signals. The present invention is primarily used in conjunction with sleep centers for the examination, diagnoses and treatment of sleep disorders.

In the present invention, a physiological data acquisition assembly comprising a head harness according to claim 1
is provided. The physiological data acquisition assembly comprises a physiological data acquisition module adapted to be removably housed by the head harness, wherein the head harness includes a base strap adapted to be adjustably secured around the circumference of a user's head, an upper portion housing the physiological data acquisition module and a plurality of longitudinally extending straps for detachably securing the housing for the physiological data acquisition module to the base strap. The physiological data acquisition assembly also comprises at least one electrode snap connector assembly for use on an active electrode and at least one electrode snap connector assembly for use on a biased ground electrode.

According to another embodiment of the present invention, a physiological data acquisition assembly is provided comprising, at least a single channel of physiological data wherein at least one active electrode and at least one reference electrode are in close proximity. The physiological data acquisition assembly also comprises a singular sensor patch wherein the at least one active electrode and at least one reference electrode are located but not electrically coupled.

According to yet another embodiment of the present invention, a system for acquiring physiological data is provided comprising a physiological data acquisition module coupled to at least one electrode snap connector assembly for use on an active electrode and at least one electrode snap connector assembly for use on a reference electrode. The system also comprises a head harness for housing the physiological data acquisition module, wherein the head harness includes a base strap adapted to be adjustably secured around the circumference of a user's head, an upper portion housing the physiological data acquisition module and a plurality of longitudinally extending straps for detachably securing the housing for the physiological data acquisition module to the base strap. The system also comprises a means for displaying, storing, processing and analyzing data transmitted by the physiological data acquisition module.

According to yet another embodiment of the present invention, a system for acquiring physiological data is provided comprising at least a single channel of physiological data wherein at least one active electrode and at least one reference electrode are in close proximity. The system also comprises a singular sensor patch wherein the at least one active electrode and the at least one reference electrode are located but not electrically coupled. The system also comprises a means for displaying, storing, processing and analyzing encrypted data transmitted by the physiological data acquisition module.

Unless otherwise defined, all terms used herein have the same meaning as commonly understood by one of ordinary skill in the art to which this invention belongs. Methods and materials are described herein for use of the present invention; other suitable methods and materials known in the art can also be used. The materials and methods, and examples are illustrative only and not intended to be limiting.

These and other embodiments of the invention will be better appreciated and understood when considered in conjunction with the following description and the accompanying drawings. It should be understood, however, that the following description, while indicating various embodiments of the invention and numerous specific details thereof, is given by way of illustration and not of limitation. Many substitutions, modifications, additions and/or rearrangements may be made within the scope of the invention as defined by the claims.

### BRIEF DESCRIPTION OF DRAWINGS

The present invention, both as to its organization and manner of operation, together with further objects and advantages, thereof, may best be understood with reference to the following description, taken in connection with the accompanying drawings in which:
FIG. 1 is a drawing of an exploded perspective view of the physiological data acquisition assembly for use in combination with a head harness.
FIG.2 is a drawing of a front perspective view of the physiological data acquisition assembly for use in combination with a head harness as applied to a patient.
FIG. 3 shows a flowchart of the system according to the present invention. The device contains the components below without limiting the general idea of the invention.

### DETAILED DESCRIPTION OF THE INVENTION

The ability to monitor physiological data for the study of brain performance during the normal course of daily activities including but not limited to sleep, in the past, has required cumbersome detection and analysis equipment and in many instances has required the need for technical assistance. The present invention disclosed herein provides a benefit over existing conventional methods by allowing the collection of data related to a physiological event from the comfort of the patient's home. The present invention employs a novel physiological data acquisition assembly and easy to follow system that is user friendly and overcomes the disadvantages of the conventional monitoring methods.

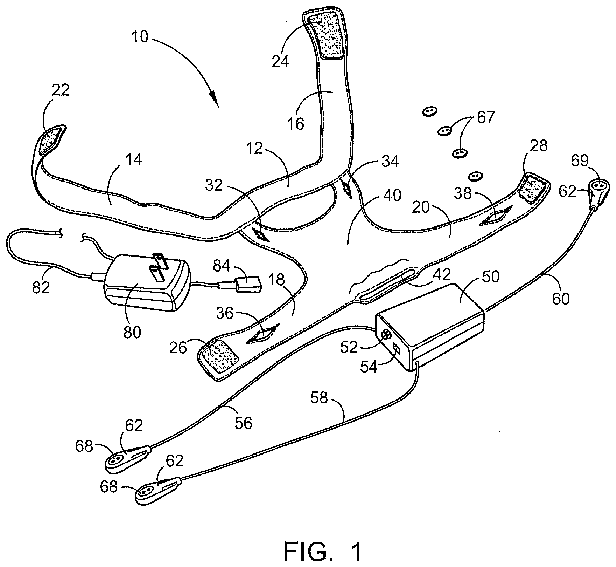

Referring to FIG. 1, an exploded perspective view of the physiological data acquisition assembly for use in combination with a head harness **10** is depicted. The head harness 10 may house one or more devices or wires. The head harness is worn over the head and hair of a patient. The head harness includes a front pad **12** having a first end **14** and second end **16.** The front pad **12** is adapted to extend across the patient's forehead. The first end 14 and second end **16** are adapted to be adjustably secured around the circumference of a user's head using a fastener preferably a hook **22** and loop **24.** In another embodiment, the fastener may be a hook and loop, Velcro, snap, button, buckle or any other fastening device that allows for custom fit, adjustment and comfort.

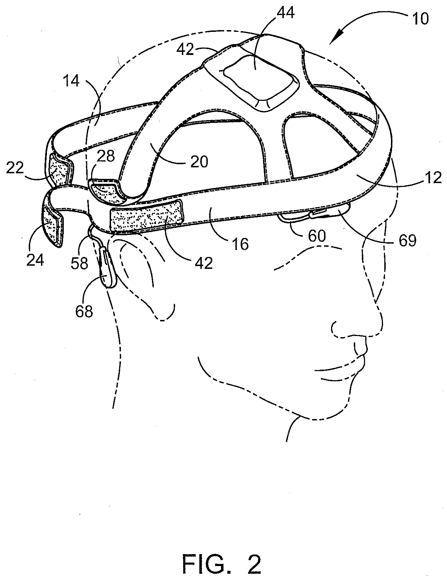

The head harness also includes an upper portion **40** including longitudinally extending straps **18** and **20** for detachably securing the upper portion **40** to the base strap **12, 14,** & **16.** The longitudinally extending straps **18** and **20** are detachably secured to the base strap at **14 & 16** using a fastener, preferably a hook **26** & **28** and loop **42,** FIG.2. In another embodiment, the fastener may be a hook and loop, Velcro, snap, button, buckle or any other fastening device that allows for custom fit, adjustment and comfort.

In a preferred embodiment, the head harness may be made of one or more layers of material to create a hollow core through all parts of the harness. According to the invention, the physiological data acquisition module **50** is removably received in the hollow core **42** & **44,** FIG. 2 of the upper portion **40.** In this preferred embodiment, the upper portion **40** and longitudinally extending straps **18** & **20** include a plurality of slots **32, 34, 36** & **38** for receiving the electrode snap connector assemblies **68** & **69** and associated lead wires **56, 58** & **60** coupled to the physiological data acquisition module **50.**

The electrode snap connector assemblies **68** represent a combination of a biased ground electrode and a reference electrode snap connectors and in the preferred embodiment the electrodes **67** are placed behind the left and right ear of the patient, FIG. 2. The biased ground electrode snap connector assembly and the reference electrode snap connector assembly **68** may be color coded to distinguish the two. In another embodiment of the present invention, only a reference electrode snap connector assembly is required for the acquisition of electrical physiological data. The electrode snap connector assembly **69** represents the active electrode snap connector and in the preferred embodiment the electrode **67** is placed on the forehead of the patient. The active electrode and reference/biased ground electrodes **67** may be a self-adhesive conductive electrode, a wet, dry, contact, non-contact or EKG electrode. The electrode snap connector assemblies also include a noise reducing or cancelling amplifier **62** at the electrode connection level to reduce any electrical noise that may be picked up by the lead wires **56, 58** & **60.** To further improve the performance of the physiological data acquisition module **50,** the module or the electrode snap connector assemblies **68** & **69** are configured to continuously monitor electrode impedance and may include lights indicative of the current status of the integrity of the electrode contacts.

**In** another embodiment of the present invention, both active and reference electrodes **67** are placed in close proximity with respect to each other but are not electrically connected. **In** this embodiment, the active and reference electrodes are located on a singular sensor patch.

In a preferred embodiment, the physiological data acquisition module **50** includes a battery power component that includes a rechargeable small form factor, high capacity battery. The physiological data acquisition module **50** includes a power supply and recharging circuitry for receiving power through an electrical power cord **82** and AC unit **80.** The electrical power cord **82** is coupled to the physiological data acquisition module for recharging the small form factor, high capacity battery through a port **54,** which may be but is not limited to USB, DB-25 or the like. The physiological data acquisition module **50** includes a power on and off function **52** for preserving the power supply of the small form factor, high capacity battery when not in use. The physiological data acquisition module **50** may also include power on and off indicator lights indicative of the current status of the physiological data acquisition module **50.** In another embodiment, the physiological data acquisition module rechargeable small form factor, high capacity battery may be recharged through a USB connection to a computer.

The physiological data acquisition module **50** is configured to record, transmit and store encrypted data collected from the electrode snap connector assembly **69** for use on an active electrode **67** applied to the forehead region of a patient. The electrode snap connector assemblies **68** for use on a reference and biased ground electrode **67** are placed behind the ears of the patient. The biased ground electrode **67** functions to stabilize the baseline and improve immunity from external interferences.

In a preferred embodiment, the physiological data acquisition module **50** is configured to include a wireless transmitter/receiver for transmitting wirelessly the recorded and stored encrypted data to a remote center or computer for further display, storing, processing and analysis or for transmitting wirelessly in real time the encrypted data to a remote center or computer for further display, storing, processing and analysis. In another aspect of the present invention, the recorded and stored data may be transmitted wirelessly to a device including but not limited to a cellular telephone, smart-phone, iPad^{®} and/or computer. The wireless transmitter/receiver may also be included on the singular sensor patch to transmit wirelessly to a device including but not limited to a cellular telephone, smart-phone, iPad^{®} and/or computer. The recorded and stored data may also be transmitted directly to a computer, cellular telephone, smart-phone and/or iPad^{®} via USB transfer capabilities incorporated at port **54** of the physiological data acquisition module **50.**

Referring now to FIG. 2, a front perspective view of the physiological data acquisition assembly for use in combination with a head harness as applied to a patient is depicted. According to the invention, the physiological data acquisition module **50** is housed in a hollow cavity **44** of the upper portion **40** of the head harness. For the purpose of illustrating the invention, in a comparative example, the physiological data acquisition module **50** is removably affixed to the head harness by a fastener that may be a hook and loop, Velcro, snap, button, buckle or any other fastening device that allows for custom fit, adjustment and comfort.

In yet another embodiment of the head harness there may be openings that allow access to the interior of the harness as well as allow for connections to be made from the interior of the harness and exterior components. In another embodiment the design may be independent of any specific device or wire purpose other than those listed here. The head harness allows any devices or wires or electronic components to be removed for service, replacement or safety. The head harness may be washable, cleaned or sterilized. The head harness may be disposable, independent of the devices or wires housed.

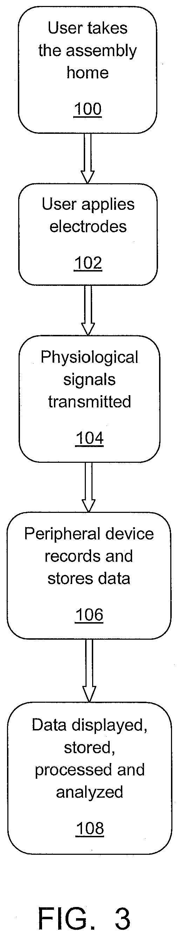

Referring now to FIG. 3, a flowchart of the system according to the present invention is depicted. In the preferred embodiment, a user is supplied with the invention and allowed to use its application in the home **100.** It is to be understood that the nature of the present invention allows the user to apply the device in any setting and is not limited to home or clinical use. At 102, due to the ease of application, the user applies at least one electrode designated the active electrode and at least one electrode designated the reference electrode. In one embodiment, the active and reference electrode may be applied to the forehead and behind the ear respectively. In another embodiment, the active electrode is applied to the forehead while a reference electrode and a biased ground electrode are applied behind the ears of the user. In yet another embodiment, the active electrode and reference electrode are contained in close proximity on a singular sensor patch and applied to the head of the user. It is to be understood that the application of the electrodes may or may not be used in combination with a head harness.

Once the electrodes have been placed by the user, physiological electrical data is collected. At **104,** the physiological data is transmitted either wirelessly by a physiological data acquisition module **50** or is transmitted wirelessly directly from the singular sensor patch to a peripheral device that may be but is not limited to a computer, cellular telephone, smart-phone and/or iPad^{®}. The peripheral device is configured to record and store the data **106.** Further, the peripheral device is configured to display, store, process and analyze **108** the transmitted encrypted data. The means for displaying, storing, processing and analyzing may be but is not limited to a computer, cellular telephone, smart-phone and/or iPad^{®} or any other remote display device.

In certain alternative embodiments of the present invention, the assembly and system are configured to accommodate more than one channel of physiological data. For example and not by way of limitation, the assembly and system incorporate sensors, i.e., head position sensor, airflow sensor using acoustics, nasal pneumotachometer, body temperature sensor and oximeter, alone or in various combinations for collecting data.

The assembly and system may also be in communication with a remote control device. The remote control device may function as a gateway device to other peripheral devices. In this capacity, the remote control device is configured to record and store encrypted data transmitted by the assembly and system, monitor the small form factor, high capacity battery life and recorded and stored data levels maintained by the physiological data acquisition module. Further, the remote control device in its capacity as a gateway device may transmit and receive recorded and stored encrypted data either through a wired or wireless connection with a peripheral device for display, storage, processing and analysis.

Systems and materials are described herein. However, systems and materials similar or equivalent to those described herein can be also used. The materials, systems, and examples are illustrative only and not intended to be limiting.

It will be apparent to those skilled in the art that various modifications and variations can be made in the present invention without departing from the scope of the invention. Other embodiments of the invention will be apparent to those skilled in the art from consideration of the specification and practice of the invention disclosed herein. It is intended that the specification and examples be considered as exemplary only, with a true scope of the invention being defined by the following claims.

## Claims

1. A physiological data acquisition assembly comprising:
a physiological data acquisition module (50);
a head harness (10) having a base strap (12, 14), a housing portion (42, 44) defining a hollow cavity (44) configured to house the physiological data acquisition module (50), and a plurality of longitudinally extending straps (18, 20) extending from the housing portion (42, 44), wherein:
the base strap (12,14) has a front pad (12) adapted to extend across a forehead of a user, and wherein the base strap has a first end (14) and a second end (16) that are adapted to be adjustably secured around the circumference of the user's head via a fastener (22, 24), and
when the head harness (10) is in use by a user, the housing portion (42, 44) is positioned above the forehead of the user by the plurality of longitudinally extending straps (18, 20) being detachably secured to the base strap (12,14);
at least one electrode snap connector assembly (68, 69) for use on an active electrode; and
at least one electrode snap connector assembly (68, 69) for use on a reference electrode.

2. The physiological data acquisition assembly of claim 1, wherein the physiological data acquisition module (50) further comprises at least one electrode snap connector assembly (68, 69) for use on a biased ground electrode.

3. The physiological data acquisition assembly of claim 2, wherein the electrode snap connector assemblies include a noise reducing or cancelling amplifier.

4. The physiological data acquisition assembly of claim 2, wherein the active electrode, reference electrode and biased ground electrode is a self-adhesive conductive wet, dry, contact or non-contact electrode.

5. The physiological data acquisition assembly of claim 1, wherein the physiological data acquisition module (50) is configured to record, transmit and store encrypted data collected from the active electrode.

6. The physiological data acquisition assembly of claim 5, wherein the physiological data acquisition module (50) includes at least one port (54) to charge the module, transmit and receive data.

7. The physiological data acquisition assembly of claim 6, wherein the physiological data acquisition module (50) further comprises a RF transmitter-receiver for transmitting encrypted data to a remote center or computer for further display, storing, processing and analysis.

8. The physiological data acquisition assembly of claim 7, wherein the recorded and stored encrypted data is transmitted wirelessly to a device selected from at least one member of the group consisting of a cellular telephone, smart-phone and computer.

9. The physiological data acquisition assembly of claim 8, wherein the RF transmitter- receiver is in wireless communication with a remote control.

10. The physiological data acquisition assembly of claim 9, wherein the remote control acts as a power source or docking station.

11. The physiological data acquisition assembly of claim 1, wherein the physiological data acquisition module (50) includes additional components selected from at least one member of the group consisting of a head positioning sensor, nasal pneumotachometer, body temperature sensor and oximeter.

12. A system for acquiring physiological data, the system comprising:
a physiological data acquisition assembly according to claim 1; and
a means for displaying, storing, processing and analyzing encrypted data transmitted by the physiological data acquisition module (50).

13. The system of claim 12, wherein the encrypted data transmitted by the physiological data acquisition module (50) is transmitted by a RF transmitter-receiver to the means for displaying, storing, processing and analysing.

## Patentansprüche

1. Anordnung zur Erfassung physiologischer Daten, umfassend:
ein Physiologische-Daten-Erfassungsmodul (50);
ein Kopfgeschirr (10) mit einem Basisriemen (12, 14), einem Gehäuseabschnitt (42, 44), der einen Hohlraum (44) definiert, welcher dazu ausgelegt ist, das Physiologische-Daten-Erfassungsmodul (50) aufzunehmen, und einer Vielzahl von sich in Längsrichtung erstreckenden Riemen (18, 20), die sich von dem Gehäuseabschnitt (42, 44) wegerstrecken, wobei:
der Basisriemen (12, 14) ein vorderes Kissen (12) aufweist, das angepasst ist, um sich quer über eine Stirn eines Benutzers zu erstrecken, und wobei der Basisriemen ein erstes Ende (14) und ein zweites Ende (16) aufweist, die angepasst sind, um über ein Befestigungselement (22, 24) um den Umfang des Kopfes des Benutzers einstellbar festgelegt zu sein, und
wenn das Kopfgeschirr (10) in Verwendung durch einen Benutzer ist, der Gehäuseabschnitt (42, 44) oberhalb der Stirn des Benutzers angeordnet ist, indem die Vielzahl von sich in Längsrichtung erstreckenden Riemen (18, 20) an dem Basisriemen (12, 14) lösbar festgelegt sind;
zumindest eine Elektrodenschnappverbinderanordnung (68, 69) zur Verwendung auf einer aktiven Elektrode; und
zumindest eine Elektrodenschnappverbinderanordnung (68, 69) zur Verwendung auf einer Bezugselektrode.

2. Anordnung zur Erfassung von physiologischen Daten nach Anspruch 1, wobei das Physiologische-Daten-Erfassungsmodul (50) ferner zumindest eine Elektrodenschnappverbinderanordnung (68, 69) zur Verwendung auf einer vorgespannten Masseelektrode umfasst.

3. Anordnung zur Erfassung von physiologischen Daten nach Anspruch 2, wobei die Elektrodenschnappverbinderanordnungen einen Geräuschminderungs- oder -unterdrückungsverstärker aufweisen.

4. Anordnung zur Erfassung von physiologischen Daten nach Anspruch 2, wobei die aktive Elektrode, die Bezugselektrode und die vorgespannte Masseelektrode eine selbstklebende, leitfähige Nass/Trocken-Kontaktelektrode oder -Nichtkontaktelektrode ist.

5. Anordnung zur Erfassung von physiologischen Daten nach Anspruch 1, wobei das Physiologische-Daten-Erfassungsmodul (50) dazu ausgelegt ist, verschlüsselte Daten, die von der aktiven Elektrode gesammelt werden, aufzuzeichnen, zu senden und zu speichern.

6. Anordnung zur Erfassung von physiologischen Daten nach Anspruch 5, wobei das Physiologische-Daten-Erfassungsmodul (50) zumindest einen Anschluss (54) zum Laden des Moduls und zum Senden und Empfangen von Daten umfasst.

7. Anordnung zur Erfassung von physiologischen Daten nach Anspruch 6, wobei das Physiologische-Daten-Erfassungsmodul (50) ferner einen HF-Sender-Empfänger zum Senden von verschlüsselten Daten an ein externes Zentrum oder einen Computer zum weiteren Anzeigen, Speichern, Verarbeiten und Analysieren umfasst.

8. Anordnung zur Erfassung von physiologischen Daten nach Anspruch 7, wobei die aufgezeichneten und gespeicherten verschlüsselten Daten drahtlos an eine Vorrichtung gesendet werden, die aus zumindest einem Element der Gruppe bestehend aus einem Mobiltelefon, einem Smartphone und einem Computer ausgewählt ist.

9. Anordnung zur Erfassung von physiologischen Daten nach Anspruch 8, wobei der HF-Sender-Empfänger in Drahtloskommunikation mit einer Fernsteuerung ist.

10. Anordnung zur Erfassung von physiologischen Daten nach Anspruch 9 wobei die Fernsteuerung als Leistungsquelle oder Andockstation fungiert.

11. Anordnung zur Erfassung von physiologischen Daten nach Anspruch 1, wobei das Physiologische-Daten-Erfassungsmodul (50) zusätzliche Komponenten ausgewählt aus zumindest einem Element der Gruppe bestehend aus einem Kopfpositionierungssensor, einem Nasenpneumotachometer, einem Körpertemperatursensor und einem Oximeter umfasst.

12. System zur Erfassung physiologischer Daten, wobei das System Folgendes umfasst:
eine Anordnung zur Erfassung von physiologischen Daten nach Anspruch 1; und
ein Mittel zum Anzeigen, Speichern, Verarbeiten und Analysieren von verschlüsselten Daten, die durch das Physiologische-Daten-Erfassungsmodul (50) gesendet werden.

13. System nach Anspruch 12, wobei die verschlüsselten Daten, die durch das Physiologische-Daten-Erfassungsmodul (50) gesendet werden, durch einen HF-Sender-Empfänger an das Mittel zum Anzeigen, Speichern, Verarbeiten und Analysieren gesendet werden.

## Revendications

1. Ensemble d'acquisition de données physiologiques comprenant :
un module d'acquisition de données physiologiques (50) ;
un harnais de tête (10) présentant une sangle de base (12, 14), une partie de logement (42, 44) définissant une cavité creuse (44) configurée pour loger le module d'acquisition de données physiologiques (50), et une pluralité de sangles s'étendant longitudinalement (18, 20) s'étendant à partir de la partie de logement (42, 44), dans lequel :
la sangle de base (12, 14) présente un patin avant (12) adapté pour s'étendre sur le front d'un utilisateur, et dans lequel la sangle de base présente une première extrémité (14) et une seconde extrémité (16) qui sont adaptées pour être fixées de manière ajustable autour de la circonférence de la tête de l'utilisateur via une fixation (22, 24), et
lorsque le harnais de tête (10) est utilisé par un utilisateur, la partie de logement (42, 44) est positionnée au-dessus du front de l'utilisateur par l'intermédiaire de la pluralité de sangles s'étendant longitudinalement (18, 20) étant fixées de manière amovible à la sangle de base (12, 14) ;
au moins un ensemble de connecteur à encliquetage d'électrode (68, 69) à utiliser sur une électrode active ; et
au moins un ensemble de connecteur à encliquetage d'électrode (68, 69) à utiliser sur une électrode de référence.

2. Ensemble d'acquisition de données physiologiques selon la revendication 1, dans lequel le module d'acquisition de données physiologiques (50) comprend en outre au moins un ensemble de connecteur à encliquetage d'électrode (68, 69) destiné à être utilisé sur une électrode de masse polarisée.

3. Ensemble d'acquisition de données physiologiques selon la revendication 2, dans lequel les ensembles de connecteur à encliquetage d'électrode incluent un amplificateur de réduction ou d'annulation de bruit.

4. Ensemble d'acquisition de données physiologiques selon la revendication 2, dans lequel l'électrode active, l'électrode de référence et l'électrode de masse polarisée sont une électrode auto-adhésive conductrice humide, sèche, de contact ou sans contact.

5. Ensemble d'acquisition de données physiologiques selon la revendication 1, dans lequel le module d'acquisition de données physiologiques (50) est configuré pour enregistrer, transmettre et stocker des données chiffrées collectées à partir de l'électrode active.

6. Ensemble d'acquisition de données physiologiques selon la revendication 5, dans lequel le module d'acquisition de données physiologiques (50) inclut au moins un port (54) pour charger le module, transmettre et recevoir des données.

7. Ensemble d'acquisition de données physiologiques selon la revendication 6, dans lequel le module d'acquisition de données physiologiques (50) comprend en outre un émetteur-récepteur RF pour transmettre des données chiffrées à un centre ou à un ordinateur distant pour un affichage, un stockage, un traitement et une analyse supplémentaires.

8. Ensemble d'acquisition de données physiologiques selon la revendication 7, dans lequel les données chiffrées enregistrées et stockées sont transmises sans fil à un dispositif sélectionné parmi au moins un membre du groupe constitué d'un téléphone cellulaire, d'un téléphone intelligent et d'un ordinateur.

9. Ensemble d'acquisition de données physiologiques selon la revendication 8, dans lequel l'émetteur-récepteur RF est en communication sans fil avec une télécommande.

10. Ensemble d'acquisition de données physiologiques selon la revendication 9, dans lequel la télécommande agit comme une source d'alimentation ou une station d'accueil.

11. Ensemble d'acquisition de données physiologiques selon la revendication 1, dans lequel le module d'acquisition de données physiologiques (50) comprend des composants supplémentaires sélectionnés parmi au moins un élément du groupe constitué d'un capteur de positionnement de tête, d'un pneumotachomètre nasal, d'un capteur de température corporelle et d'un oxymètre.

12. Système pour acquérir des données physiologiques, le système comprenant :
un ensemble d'acquisition de données physiologiques selon la revendication 1 ; et
des moyens pour afficher, stocker, traiter et analyser des données chiffrées transmises par le module d'acquisition de données physiologiques (50).

13. Système selon la revendication 12, dans lequel les données chiffrées transmises par le module d'acquisition de données physiologiques (50) sont transmises par un émetteur-récepteur RF aux moyens d'affichage, de stockage, de traitement et d'analyse.
